Europäisches Patentamt

European Patent Office

Office européen des brevets

(19)

(11) Veröffentlichungsnummer: **0 1 1 4 1 99**
**B1**

(12)

# EUROPÄISCHE PATENTSCHRIFT

(45) Veröffentlichungstag der Patentschrift:
07.01.88

(21) Anmeldenummer: 83110525.9

(22) Anmeldetag: 21.10.83

(51) Int. Cl.⁴: **A 61 K 31/645**, A 61 K 47/00,
A 61 K 9/20

(54) Galenische Form für Amitriptylinoxid und Verfahren zur Herstellung.

(30) Priorität: 23.12.82 DE 3247676

(43) Veröffentlichungstag der Anmeldung:
01.08.84 Patentblatt 84/31

(45) Bekanntmachung des Hinweises auf die Patenterteilung:
07.01.88 Patentblatt 88/1

(84) Benannte Vertragsstaaten:
AT BE CH DE FR GB IT LI LU NL SE

(56) Entgegenhaltungen:
FR - M - 4 832
US - A - 3 705 942
US - A - 4 287 214

(73) Patentinhaber: A. Nattermann & Cie. GmbH,
Nattermannallee 1, D-5000 Köln 30 (DE)

(72) Erfinder: Dürr, Manfred, Dr., Dipl.-Chem., Am blauen
Stein 10, D-5024 Pulheim-Dansweiler (DE)
Erfinder: Gajdos, Benedikt, Dr.,
Waffenschmidtstrasse 2, D-5000 Köln 71 (DE)
Erfinder: Gneuss, Klaus-Dieter, Dr. Dipl.-Chem.,
Siegstrasse 8, D-5000 Köln 40 (DE)
Erfinder: Harhausen, Ekkehard, Dr., Käulchensweg 32,
D-5000 Köln 91 (DE)
Erfinder: Seidel, Jürgen, Dr., Venloer Strasse 67,
D-5024 Pulheim (DE)

(74) Vertreter: Sternagel, Hans-Günther, Dr. et al,
Patentanwälte Dr. Michael Hann Dr. H.-G. Sternagel
Sander Aue 30, D-5060 Bergisch Gladbach 2 (DE)

## Beschreibung

Die Erfindung betrifft neue stabile galenische Zubereitungen für Amitriptylinoxid-dihydrat sowie ein Verfahren zu ihrer Herstellung sowie einen Amitriptylinoxiddihydrat-Zitronensäurekomplex.

Aus US-A-4 287 214 ist es bekannt, Anthralin für pharmazeutische Zwecke mittels spezieller α-Hydroxycarbonsäuren gegen Oxidation durch Luftsauerstoff zu schützen. Es wird jedoch darauf hingewiesen, dass Ascorbinsäure, Salicylsäure, Milchsäure, Glykolsäure, Mandelsäure keine ausreichende Stabilisierung bewirken.

In DE-A-15 43 224 ist die Herstellung von Dibenzocycloheptenderivaten wie Amitriptylinoxiddihydrat und ihre Verwendung als Arzneimittel beschrieben. In der Druckschrift sind keine Angaben über die Stabilität der Verbindungen enthalten. Die mit üblichen Hilfsstoffen hergestellten Präparate werden zur Therapie von Depressionen empfohlen.

Die im Handel befindlichen Formulierungen von pharmazeutischen Präparaten mit Amitriptylinoxid-dihydrat zeigen jedoch oft keine ausreichende Stabilität, insbesondere im wärmeren Klimazonen.

Aufgabe der Erfindung ist es, eine galenische Zubereitung zu schaffen, die auch unter ungünstigen Lagerbedingungen eine ausreichende Langzeitstabilität aufweist.

Diese Aufgabe wird gelöst durch eine galenische Zubereitung von Amitriptylinoxid-dihydrat und üblichen Hilfs- oder Füllstoffen, die als Stabilisator die α-Hydroxycarbonsäure Äpfelsäure, Weinsäure oder Zitronensäure enthält.

Zur Herstellung der neuen galenischen Formen wird Amitriptylinoxid-dihydrat zusammen mit der Hydroxycarbonsäure im Molarverhältnis von 1:1 bis 1:2 in Wasser oder einem Alkohol/Wasser-Gemisch gelöst. Das Amitriptylinoxid-dihydrat und die Hydroxycarbonsäure können auch getrennt in Wasser oder einem Alkohol/Wasser-Gemisch gelöst und dann zusammen gemischt werden. Als Alkohole kommen niedere aliphatide Alkohole insbesondere Isopropanol in Frage. Die erhaltene Lösung wird auf einen Hilfsstoff bzw. Träger aufgetragen, das Lösungsmittel verdampft und das erhaltene Amitriptylinoxid-dihydrat-Säure-Copräparat zu Tabletten verpresst oder in Kapseln abgefüllt. Es wird angenommen, dass sich ein Komplex aus Säure und Dihydrat bildet. Die Herstellung des Copräparates kann durch Feuchtgranulation mit anschliessender Trocknung, Wirbelschichtgranulation oder durch Aufsprühen der Lösung auf Pellets durchgeführt werden.

Als Hilfs- oder Trägerstoffe kommen übliche galenische Stoffe in Frage, z.B. Kieselgele, Ca-Phosphate, Ca-Sulfat, Monosaccharide, Oligosaccharide und Polysaccharide, Saccharose, Fructose, Reis-, Mais-, Kartoffel-Stärke, Cellulose, Faserstoffe, Polymere usw. in Frage.

Die Copräparate können auch hergestellt werden, indem man Amitriptylinoxid-dihydrat in kristalliner Form mit üblichen Hilfs- und/oder Trägerstoffen mischt, mit einer Lösung der Hydroxycarbonsäure granuliert und weiterverarbeitet.

Mit den neuen Copräparaten wurde ein Stabilitätstest durchgeführt (s. Tab. 1), wobei die Präparate einer erhöhten Temperatur ausgesetzt wurden. (Die Befunde dieses Tests lassen auf eine Lagerstabilität von mindestens 3 Jahren für die Klimazonen I–IV schliessen).

Tabelle 1

| Vers. Nr. | Wirkstoff/Säure in Gramm (jeweils mit Hilfsstoffen auf 100 g aufgefüllt) | | | Präparat | Stabilität[1] |
|---|---|---|---|---|---|
| 1 | 100,0 | – | | P | ≤ 1 |
| 2 | 13,3 | – | | T | ≤ 2 |
| 3 | 9,2 | 7,5 | Äpfelsäure | G | 15 |
| 4 | 9,2 | 7,5 | Äpfelsäure | V | 10 |
| 5 | 9,4 | 4,3 | Weinsäure | G | 15 |
| 6 | 9,4 | 4,3 | Weinsäure | V | 7 |
| 7 | 8,8 | 11,2 | Zitronen- | G | >40 |
| 8 | 8,8 | 11,2 | säuremono- | V | 10 |
| 9 | 13,3 | 17,0 | hydrat | T | >38 |

[1] Stabilität: Zeitraum in Tagen bis 10% des Wirkstoffes bei 61°C zersetzt sind

P = Pulver

T = Tablette

G = Granulat: Wirkstoff und Säure in Lösung mit Hilfsstoff feucht granuliert

V = Verreibung: die fein pulverisierten Inhaltsstoffe werden in einem Mörser oder einer Reibschale homogen gemischt

Beispiel 1

800 g Amitriptylinoxid-dihydrat und 1020,80 g Citronensäuremonohydrat werden in 1600 ml eines Isopropanol/Wasser-Gemisches (1:1, v/v) gemeinsam gelöst. Diese Lösung wird in einem Mischer (Diosna-Mischer) zu einer Mischung aus 3600 g Avicel PH (mikrokristalline Cellulose) und 120 g Aerosil 200 (Kieselsäure) gegeben. Das Feuchtgranulat wird danach getrocknet (Horden- oder Wirbelschichttrocknung). Das trockene Granulat wird im Mischer zur Herstellung der pressfertigen Mischung mit 120 g Aerosil 200, 1699,20 g Avicel PH, 102,56 g Primogel (Carboxymethylcellulose) und 80 g Magnesiumstearat gemischt. Im Anschluss daran wird die pressfertige Mischung nach üblichen Verfahren zu Tabletten verpresst.

Beispiel 2
800 g Amitriptylinoxid-dihydrat und
651,20 g DL-Äpfelsäure
werden in 4000 ml Ethanol (96%ig) gelöst. Der Lösung werden 400 g Aerosil 200 zugefügt. Dieses Gemisch wird sprühgetrocknet.

1851,20 g dieses Sprühproduktes werden mit
4708,80 g Dicalciumphosphatdihydrat
800 g Avicel PH 102
560 g Primogel und
80 g Magnesiumstearat
gemischt und die pressfertige Mischung nach üblichen Verfahren zu Tabletten verpresst.

**Patentansprüche**

1. Galenische Zubereitung von Amitriptylinoxid-dihydrat und üblichen Hilfs- oder Füllstoffen, dadurch gekennzeichnet, dass als Stabilisator die α-Hydroxycarbonsäure Äpfelsäure, Weinsäure oder Zitronensäure enthalten ist.

2. Galenische Zubereitung nach Anspruch 1, dadurch gekennzeichnet, dass das Molverhältnis von Amitriptylinoxid-dihydrat und der Hydroxycarbonsäure 1:1 bis 1:2 beträgt.

3. Verfahren zur Herstellung galenischer Zubereitungen gemäss Anspruch 1 oder 2, dadurch gekennzeichnet, dass man Amitriptylinoxid-dihydrat zusammen mit der Hydroxycarbonsäure in Wasser oder einem Alkohol-Wasser-Gemisch löst oder dass man Amitriptylinoxid-dihydrat und die Hydroxycarbonsäure jeweils getrennt in Wasser oder einem Alkohol-Wasser-Gemisch läst und die beiden Lösungen zusammenmischt, die Lösung bestehend aus Amitriptylinoxid-dihydrat und Hydroxycarbonsäure auf einen Hilfsstoff bzw. einen Träger aufträgt, das Lösungsmittel abzieht, das erhaltene Produkt gegebenenfalls granuliert und das Granulat zu Tabletten verpresst oder in Kapseln abfüllt.

4. Verfahren nach Anspruch 3, dadurch gekennzeichnet, dass man Amitriptylinoxid-dihydrat und Zitronensäure im Molverhältnis von 1:1 in Wasser oder einem Lösungsmittel-Gemisch aus Isopropanol und Wasser im Volumenverhältnis von 1:1 löst.

5. Amitriptylinoxid-dihydrat-Zitronensäure-Komplex.

**Claims**

1. Galenic preparation of amitryptyline oxide dihydrate and conventional auxiliaries or extenders, characterized in that it comprises as stabilizer the α-hydroxy carboxylic acid malic acid, tartaric acid or citric acid.

2. Galenic preparation of claim 1, characterized in that the molar ratio of amitryptyline oxide dihydrate to hydroxy carboxylic acid is 1:1 to 1:2.

3. Method for the manufacture of galenic preparations according to claim 1 or 2, characterized in that amitryptyline oxide dihydrate is dissolved jointly with the hydroxy carboxylic acid in water or an alcohol water mixture or that the amitryptyline oxide dihydrate and the hydroxy carboxylic acid are each dissolved separately in water or in an alcohol water mixture and the two solutions are mixed together, the solution consisting of amitryptyline oxide dihydrate and hydroxy carboxylic acid being applied onto an auxiliary agent respectively onto a carrier, the solvent being evaporated, the resulting product being optionally granulated and the granulate being pressed into tablets or filled into capsules.

4. The method of claim 3, characterized in that the amitryptyline oxide dihydrate and citric acid are dissolved in a molar ratio of 1:1 in water or a solvent mixture of isopropanol and water in volume ratio of 1:1.

5. A complex of amitryptyline oxide dihydrate and citric acid.

**Revendications**

1. Préparation galénique d'oxyde d'amitriptyline dihydrate et de substances auxiliaires ou de charges usuelles, caractérisée en ce qu'elle contient, comme stabilisant, l'acide α-hydroxycarboxylique acide malique, acide tartrique ou acide citrique.

2. Préparation galénique selon la revendication 1, caractérisée en ce que le rapport molaire de l'oxyde d'amitriptyline dihydrate et de l'acide hydroxycarboxylique est compris entre 1:1 et 1:2.

3. Procédé d'obtention de préparations galéniques selon l'une des revendications 1 ou 2, caractérisé en ce qu'on dissout de l'oxyde d'amitriptyline dihydrate en même temps que l'acide hydroxycarboxylique dans de l'eau ou un mélange eau-alcool, ou bien qu'on dissout séparément chacun des produits oxyde d'amitriptyline dihydrate et acide hydroxycarboxylique dans de l'eau ou un mélange eau-alcool et qu'on mélange ensemble les deux solutions; qu'on dépose sur une substance auxiliaire ou un support la solution constituée d'oxyde d'amitriptyline dihydrate et d'acide hydroxycarboxylique; qu'on élimine le solvant, qu'on réalise éventuellement une granulation du produit obtenu et qu'on comprime le granulé sous forme de comprimés ou qu'on en remplit des capsules.

4. Procédé selon la revendication 3, caractérisé en ce qu'on dissout de l'oxyde d'amitriptyline dihydrate et de l'acide citrique dans un rapport molaire de 1:1, dans de l'eau ou un mélange solvant d'eau et d'isopropanol dans un rapport volumique 1:1.

5. Complexe d'oxyde d'amitriptyline dihydrate et d'acide citrique.